# EUROPÄISCHE PATENTSCHRIFT

(11) **EP 1 511 435 B1**
(45) Veröffentlichungstag und Bekanntmachung des Hinweises auf die Patenterteilung: **28.05.2008**
(21) Anmeldenummer: 03735310.9
(22) Anmeldetag: 19.05.2003
(51) Int. Cl.: A61B 17/70

(54) **VERANKERUNGSELEMENT ZUR BEFESTIGUNG EINES STABES EINER VORRICHTUNG ZUM EINRICHTEN EINER MENSCHLICHEN ODER TIERISCHEN WIRBELSÄULE AN EINEM WIRBELKNOCHEN**
ANCHORING ELEMENT FOR SECURING A ROD OF A DEVICE FOR ADJUSTING A HUMAN OR ANIMAL VERTEBRAL COLUMN ON A VERTEBRA
ELEMENT D'ANCRAGE POUR FIXER UNE TIGE D'UN DISPOSITIF SERVANT A AJUSTER UNE COLONNE VERTEBRALE HUMAINE OU ANIMALE SUR UNE VERTEBRE

(30) Priorität: 21.05.2002 DE 20207851 U
(43) Veröffentlichungstag der Anmeldung: 09.03.2005
(73) Patentinhaber: Metz-Stavenhagen, Peter, 34537 Bad Wildungen (DE)
(72) Erfinder: Metz-Stavenhagen, Peter, 34537 Bad Wildungen (DE)
(74) Vertreter: Walther, Walther & Hinz GbR
(86) Internationale Anmeldenummer: PCT/DE2003/001609
(87) Internationale Veröffentlichungsnummer: WO 2003/096915

(56) Entgegenhaltungen:
- EP-A- 1 023 873
- US-A- 5 190 543
- US-A- 5 591 166
- US-A- 5 879 350

## Beschreibung

Die vorliegende Erfindung betrifft ein Verankerungselement zur Befestigung eines Stabes einer Vorrichtung zum Einrichten einer menschlichen oder tierischen Wirbelsäule an einem Wirbelknochen, mit einer Halterung zur Aufnahme des Stabes, mit einem gegen den Stab wirkenden und an der Halterung anbringbaren Sicherungselement, mit einem Befestigungselement zur Anbringung am Wirbelkörper und mit einer zwischen der Halterung und dem Befestigungselement angeordneten Klemmvorrichtung, umfassend eine ringförmige Einfassung, ein teilweise kugelsegmentförmiges Lager und ein das Lager umgreifendes und in die Einfassung eingelassenes Zwischenelement, wobei die Einfassung in gelöstem Zustand gegenüber dem Lager frei beweglich ist, während die Einfassung in geklemmtem Zustand über das Zwischenelement klemmend am Lager gehalten ist und wobei die Einfassung starr mit der Halterung und das Lager starr mit dem Befestigungselement verbunden ist.

Aus der EP 0 885 598 A2 ist ein als Pedikelschraube ausgeführtes Verankerungselement mit den oben genannten Merkmalen bekannt. Dabei ist das Befestigungselement als Gewindeschaft ausgebildet, an dessen Kopfende ein Lager ausgebildet ist. Dieses Lager ist einstückig mit dem Gewindeschaft verbunden und in Umfangsrichtung kugelförmig ausgebildet. Das Lager wird von einem Zwischenelement umgeben, welches in Umfangrichtung am Lager anliegt. Dabei sind die Gegenflächen des Zwischenelementes derart gekrümmt ausgebildet, dass diese passgenau am Lager zur Anlage kommen. Das Zwischenelement besitzt über den Umfang verteilt drei axial ausgerichtete Schlitze, so dass die zwischen den Schlitzen entstehenden Segmente radial verschiebbar sind. Das Zwischenelement ist in einer Einfassung gehalten, die sich zum Gewindeschaft hin verjüngt, so dass das Zwischenelement nicht nach unten herausfallen kann. An der Einfassung ist eine U-förmige Halterung zur Aufnahme eines Verbindungs-, Kompressions- oder Distraktionsstabes einstückig angeformt. In den beiden Haltestegen der Halterung ist innenliegend ein Sägezahngewinde vorgesehen, in das ein als Madenschraube ausgeführtes Sicherungselement zur Fixierung des Stabes einsetzbar ist. Das Zwischenelement ist dabei derart ausgelegt, dass sein oberer Bereich in den Aufnahmeschlitz der Halterung hineinreicht. Unter einem Sägezahngewinde sind neben dem metrischen Sägengewinde gemäß DIN 513 auch Sägezahngewinde mit einem etwas größeren oder etwas kleineren Flankenwinkel, mit einem Flankenwinkel von 0° oder einem negativen Flankenwinkel, sowie Sägezahngewinde gemäß EP 885 598 zu verstehen.

Auch aus der DE 39 23 996 C2, DE 100 05 385 A1, EP 1 090 595 A2 und WO 98/25534 A1 sind Verankerungselemente bekannt, bei denen Pedikelschrauben kugelsegmentförmige Kopfenden aufweisen, die in einer entsprechenden Halterung klemmbar gehalten sind.

Bei der Implantation dieser Verankerungselemente wird zunächst der Gewindeschaft in den entsprechenden Wirbelkörper eingeschraubt. Anschließend wird der Stab in den Aufnahmeschlitz eingelegt und mit einer Madenschraube gehalten. Beim weiteren Anziehen der Madenschraube drückt der Stab auf das Zwischenelement, so dass die Einfassung relativ zum Zwischenelement verschoben wird. Aufgrund der sich verjüngenden Ausgestaltung der Einfassung werden dabei die Segmente des Zwischenelementes radial zusammengedrückt und üben eine Haltekraft auf das Lager aus. Das heißt mit anderen Worten, im gelösten Zustand kann die Einfassung mit der Halterung relativ zum Lager bzw. zum Gewindeschaft frei bewegt werden, während im angezogenen Zustand die Einfassung aufgrund der Klemmwirkung fest und unbeweglich am Lager gehalten ist. Dabei kann die Einfassung bzw. die Halterung relativ zum Lager um die Längsachse rotiert werden und die Einfassung bzw. Halterung kann entsprechend dem Kreissegment des Lagers quer zur Längsachse geschwenkt werden. Hierdurch ist es möglich, zunächst einmal die Pedikelschraube im Wirbelknochen zu verankern und anschließend die Halterung in der gewünschten Weise auszurichten, um den Stab in der optimalen Stellung zum Wirbelkörper zu fixieren.

Bei derartigen Verankerungselementen ist die Halterung mittels Kraftschluss am Schaft gehalten. Beim Einsatz dieser Verankerungselemente an der vendralen Wirbelsäule kommt es jedoch vor, dass die vom Stab auf den Wirbelkörper zu übertragenden Kräfte sehr groß sind und dass der hier vorhandene Kraftschluss diese großen Kräfte nicht übertragen kann. In diesen Fällen verrutscht die Halterung und der Stab ist nicht mehr in der gewünschten Position.

Davon ausgehend liegt der vorliegenden Erfindung die Aufgabe zugrunde, ein Verankerungselement der eingangs genannten Art zu schaffen, welches trotz beweglich angebrachter Halterung in der Lage ist, relativ große Kräfte vom Stab auf den Wirbelkörper zu übertragen, ohne zu verrutschen.

Als technische Lösung dieser Aufgabe wird erfindungsgemäß vorgeschlagen, dass eingangs genannte Verankerungselement gemäß den Merkmalen des Anspruchs 1 weiterzubilden. Vorteilhafte Weiterbildungen sind den Unteransprüchen zu entnehmen.

Ein nach dieser technischen Lehre ausgebildetes Verankerungselement hat den Vorteil, dass die Einfassung und die Halterung nach wie vor relativ zum Lager und zum Schaft beweglich sind und dass dennoch aufgrund der ausgebildeten Führungsflächen und der dazu korrespondierenden Gegenflächen die auftretenden Kräfte formschlüssig übertragen werden. Bei dieser Ausgestaltung kann die Einfassung und die Halterung im Vergleich zum Stand der Technik lediglich in einer Richtung quer zur Längsachse bewegt werden. Aus diesem Grunde könnte man das erfindungsgemäße Verankerungselement als monoaxiale Schraube bezeichnet, während das Verankerungselement gemäß dem Stand der Technik wegen seiner Beweglichkeit in alle Richtungen als polyaxiale Schraube benannt wird.

Ein weiterer Vorteil des erfindungsgemäßen Verankerungselementes besteht darin, dass wegen der formschlüssigen Übertragung der Kräfte nunmehr die benötigte Klemmfläche deutlich verringert werden kann, da kein so starker Reibschluss mehr erzeugt werden braucht. Aus diesem Grunde kann das erfindungsgemäße Verankerungselement sehr viel kleiner ausgeführt werden. Dieses kleine Verankerungselement kann dann vorteilhaft im Halswirbelbereich eingesetzt werden.

In bevorzugten Ausführungsformen sind die Führungsflächen und/oder die Gegenflächen geriffelt oder aufgeraut ausgebildet. Hierdurch erhöht sich die Reibung der beiden Flächen zueinander mit der Folge, dass die zur Fixierung der Einfassung bzw. Halterung erforderlichen Kräfte deutlich geringer sein können. Auch dieses Merkmal trägt im Ergebnis zu einer Verkleinerung des gesamten Verankerungselementes bei, da zur ausreichenden Klemmung nunmehr eine geringe Fläche benötigt wird.

In einer anderen, bevorzugten Ausführungsform ist in den Führungsflächen und in den Gegenflächen eine Rastvorrichtung ausgebildet. Diese weist insbesondere in den Führungsflächen und in den Gegenflächen radial angeordnete Nuten auf, die derart voneinander beabstandet sind, dass die Gegenfläche in gegenüberliegende Nuten der Führungsfläche eingreift und umgekehrt. Durch dieses passgenaue Eingreifen der beiden Flächen kann die Halterung bzw. Einfassung in den vorgegebenen Raststufen beim Implantieren bereits grob eingestellt werden, bevor der Stab eingelegt wird. Bevor die Sicherungsschraube dann festgezogen wird, ist selbstverständlich eine Feinjustierung noch möglich. Diese Rasterung erleichtert das Implantieren des Verankerungselementes und ermöglicht eine weitergehende Verkleinerung desselben, da die Halterung bzw. Einfassung durch die Rasterung bereits fixiert wird, so dass eigentliche Klemmfläche noch weiter verkleinert werden kann.

In einer weiteren, bevorzugten Ausführungsform ist das Lager zumindest in seinem kugelsegmentförmigen Bereich und/oder in den diesem Bereich zugewandten Halteflächen des Zwischenelementes aufgeraut ausgebildet. Durch diese Aufrauung wird die Klemmung der Einfassung am Lager verbessert, so dass bei gleicher Fläche eine bessere Klemmwirkung erreicht wird.

Weitere Vorteile des erfindungsgemäßen Verankerungselementes ergeben sich aus der beigefügten Zeichnung und den nachfolgend beschriebenen Ausführungsformen. Ebenso können die vorstehend genannten und die noch weiter ausgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter. Es zeigen:
- Fig. 1: eine Frontansicht eines erfindungsgemäßen Verankerungselementes mit einem explosionsartig herausgezogenen Sicherungselement;
- Fig. 2a, 2bdas: Verankerungselement gemäß Fig. 1, geschnitten entlang Linie II - II;
- Fig. 3: eine Draufsicht auf das Verankerungselement gemäß Fig. 1, geschnitten entlang Linie III - III;
- Fig. 4a, 4b eine: perspektivische Teilansicht der Verankerungselemente gemäß Fig. 2a und 2b;
- Fig. 5: eine perspektivische Ansicht des Zwischenelementes des Verankerungselementes gemäß Fig. 1;
- Fig. 6: eine perspektivische Ansicht des Lagers des Verankerungselementes gemäß Fig. 1.

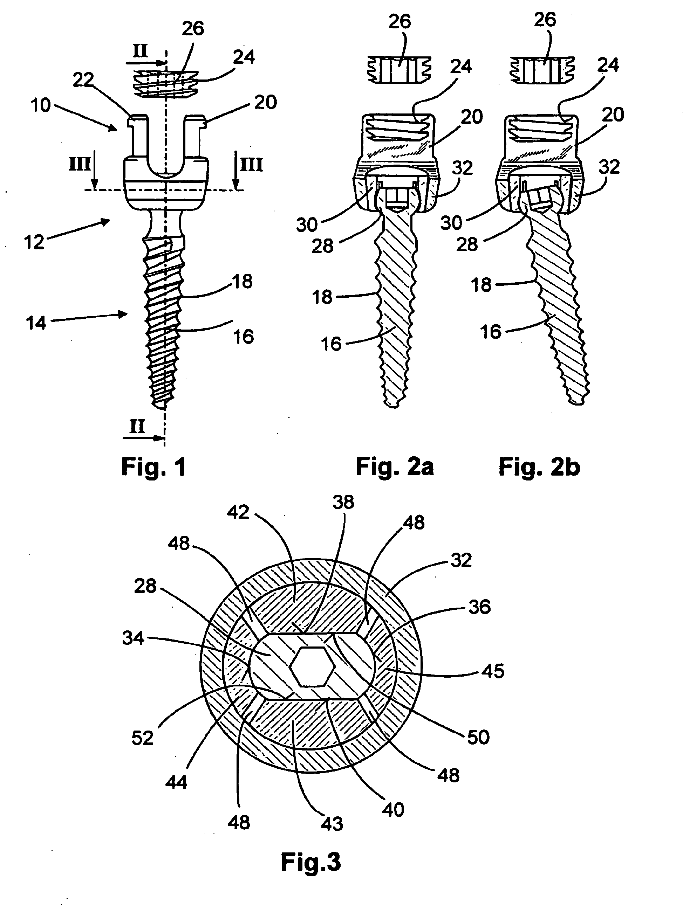

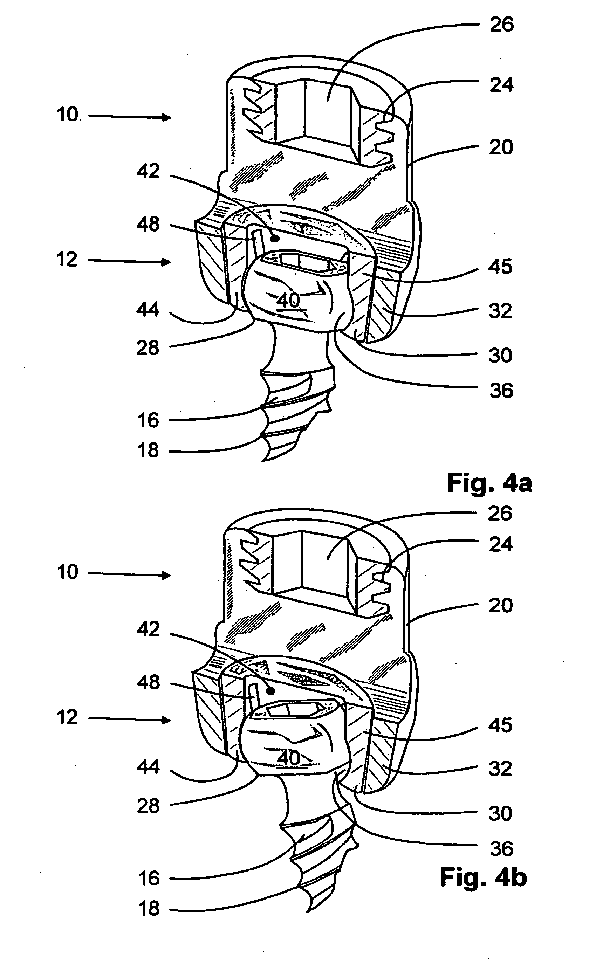

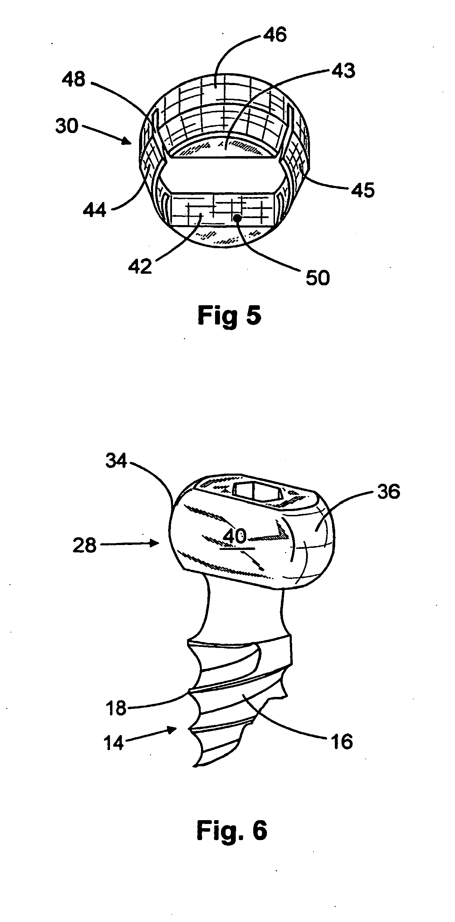

Das in den Figuren 1 bis 6 dargestellte Verankerungselement ist Bestandteil einer Vorrichtung zum Einrichten einer menschlichen oder tierischen Wirbelsäule, wie es beispielsweise unter dem Handelsnamen XIA der Firma Stryker angeboten wird. Dieses Verankerungselement dient der Befestigung eines längeren Stabes an einem Wirbelknochen und umfasst eine Halterung 10 zur Aufnahme eines hier nicht dargestellten Stabes, eine Klemmvorrichtung 12 und ein Befestigungselement 14. Das Befestigungselement 14 ist aus einem sich konisch verjüngenden Schaft 16 gebildet, an dem beispielsweise ein Biogewinde 18, ein Spongiosagewinde oder dergleichen aufgebracht ist. Mit dem Befestigungselement 14 wird das Verankerungselement in den Wirbelkörper eingebracht und fixiert.

Die Halterung 10 ist U-förmig ausgebildet und umfasst zwei koaxial angeordnete Stege 20, 22, an deren Innenseite ein Sägezahngewinde 24 ausgebildet ist. Dieses Sägezahngewinde 24 ist detailliert in der EP 0 885 598 A2 beschrieben. In dieser Halterung 10 kann in axialer Richtung ein als Madenschraube ausgeführtes Sicherungselement 26 eingeschraubt werden, um den in die Halterung 10 eingelegten Stab zu fixieren.

Die Klemmvorrichtung 12 umfasst ein in axialer Verlängerung des Schaftes 16 und einstückig mit diesem verbundenes Lager 28, ein das Lager 28 umgreifendes Zwischenelement 30 und eine das Zwischenelement 30 und eine das Zwischenelement 30 umfassende Einfassung 32. Das Lager 28 ist an zwei sich gegenüberliegenden Seiten 34, 36 kugelsegmentförmig ausgebildet und einstückig mit dem Schaft 16 verbunden. Zwischen den Seiten 34, 36 sind zwei plan ausgebildete Führungsflächen 38, 40 vorgesehen, die ebenfalls sich gegenüberliegend angeordnet sind.

Das im wesentlichen ringförmige Zwischenelement 30 weist in seinem das Lager 28 umgreifenden Bereich zwei sich gegenüberliegende Flachsegmente 42, 43 und zwei sich gegenüberliegende Kurvensegmente 44, 45 auf, die an einem umlaufenden Ring 46 gehalten sind. Zwischen den Flach- und Kurvensegmenten 42, 43, 44, 45 ist jeweils ein Spalt 48 ausgebildet. Dabei legen sich die Flachsegmente 42, 43 mit ihrer Gegenfläche 50, 52 bündig an die plan ausgebildete Führungsflächen 38, 40 an, während sich die Kurvensegmente 44, 45 sich bündig an die teilkreisförmig ausgebildeten Seiten 34, 36 anfügen. Nach außen hin sind die Flach- und Kurvensegmente 42, 43, 44, 45 kreisrund ausgebildet und werden passgenau, jedoch mit Spiel in der Einfassung 32 aufgenommen. Die Einfassung 32 wiederum ist einstückig mit der Halterung 10 verbunden.

Im nichtimplantierten Zustand lässt sich die Einfassung 32 zusammen mit der Halterung 10 um die Längsachse des Verankerungselementes drehen. Gleichzeitig kann die Einfassung 32 bzw. Halterung 10 parallel zu den Führungs- 38, 40 und Gegenflächen 50, 52 geschwenkt werden. Durch Einbringen des hier nicht dargestellten Stabes in die U-förmige Halterung 10 legt sich der Stab auf das in den U-förmigen Schlitz hineinreichende Zwischenelement auf und wird durch das Anziehen des Sicherungselementes 26 relativ zur Einfassung 32 bzw. Halterung 10 verschoben, so dass sich das Zwischenelement zwischen der Einfassung 32 und dem Lager 28 verklemmt, um die Halterung 10 auf dem Schaft 16 zu fixieren. Dabei ist das Verankerungselement derart in den Wirbelknochen einzuschrauben, dass die Führungsflächen 38, 40 möglichst quer zur Richtung des zu erwartenden Stabes angeordnet sind. Hierdurch können die vom Stab ausgehenden Kräfte optimal über die Führungsflächen 38, 40 in den Schaft 16 und in den Wirbelknochen eingeleitet werden.

In einer weiteren, hier nicht dargestellten Ausführungsform ist auf den Führungsflächen 38, 40 und den damit korrespondierenden Gegenflächen 50, 52 ein Raster ausgebildet, bei dem radial verlaufende Nuten in jede der sich gegenüberliegenden Flächen eingebracht sind. Dabei nehmen die Nuten der einen Fläche die Erhöhungen der gegenüberliegenden Fläche auf, so dass die Führungsflächen 38, 40 gezielt in die Gegenflächen 50, 52 eingreifen. Hierdurch ist eine Rastvorrichtung geschaffen, mit der die Halterung 10 in beliebigen Schritten vorjustiert werden kann, so dass das eigentliche Anbringen des Stabes zügig und ohne Verzögerung vorangehen kann.

In einer anderen, hier nicht dargestellten Ausführungsform sind sowohl die Führungsflächen 38, 40, als auch die Gegenflächen 50, 52 aufgeraut ausgebildet, um eine bessere Klemmwirkung zu erreichen.

Die Implantation des erfindungsgemäßen Verankerungselementes und der Vorrichtung zum Einrichten einer menschlichen oder tierischen Wirbelsäule verläuft wie folgt:

Zunächst einmal wird der Schaft 16 mit dem daran angebrachten Lager 28 in den betreffenden Wirbelknochen eingedreht. Dabei ist es empfehlenswert, die Führungsflächen 38, 40 quer zu der später beabsichtigten Stabrichtung anzuordnen. Danach wird die Halterung 10 zunächst einmal grob in die gewünschte Position gebracht, bevor das Sägezahngewinde 24 in die U-förmige Halterung 10 eingebracht wird und mit dem Sicherungselement 26 fixiert wird. Dabei drückt das Sicherungselement 26 auf den Zwischenring 30 und hebt gleichzeitig die Einfassung 32 entgegen dem Zwischenring 30 an, so dass sich der Zwischenring 30 zwischen der Halterung 10 und dem Lager 28 verklemmt. In dieser Position fixiert die Klemmvorrichtung das Verankerungselement dauerhaft, während über die Führungs- 38, 40 und die Gegenflächen 50, 52 die Kräfte vom Stab in den Schaft 16 und weiter in den Wirbelknochen eingeleitet werden. In einer anderen, bevorzugten Ausführungsform sind die einzelnen Flächen aufgeraut ausgebildet und bewirken damit eine bessere Klemmung.

Durch die kraftmäßige Trennung von der eigentlichen Klemmkraft und der zu übertragenden Kraft wird die zur Erreichung der Klemmung erforderliche Fläche deutlich reduziert, so dass das gesamte Verankerungselement sehr viel kleiner ausgeführt werden kann bzw. dass bei gleicher Größe entsprechend größere Kräfte übertragen werden können. Die kleineren Verankerungselemente können dann beispielsweise im Bereich der Halswirbel oder an anderen Stellen eingesetzt werden, in denen ansonsten kein ausreichender Platz vorhanden ist.

### Bezugszeichenliste:

- 10: Halterung
- 12: Klemmvorrichtung
- 14: Befestigungselement
- 16: Schaft
- 18: Gewinde
- 20: Steg
- 22: Steg
- 24: Sägezahngewinde
- 26: Sicherungselement
- 28: Lager
- 30: Zwischenelement
- 32: Einfassung
- 34: Seite
- 36: Seite
- 38: Führungsfläche
- 40: Führungsfläche
- 42: Flachsegment
- 43: Flachsegment
- 44: Kurvensegment
- 45: Kurvensegment
- 46: Ring
- 48: Spalt
- 50: Gegenfläche
- 52: Gegenfläche

## Patentansprüche

1. Verankerungselement zur Befestigung eines Stabes einer Vorrichtung zum Einrichten einer menschlichen oder tierischen Wirbelsäule an einem Wirbelknochen, mit einer Halterung (10) zur Aufnahme des Stabes, mit einem gegen den Stab wirkenden und an der Halterung anbringbaren Sicherungselement (26), mit einem Befestigungselement (14) zur Anbringung am Wirbelkörper und mit einer zwischen der Halterung (10) und dem Befestigungselement (14) angeordneten Klemmvorrichtung (12) umfassend eine ringförmige Einfassung (32), ein Lager (28) und ein das Lager umgreifendes und in die Einfassung (32) eingelassenes Zwischenelement (30), wobei das Lager (28) an zwei sich gegenüberliegenden Seiten (34, 36) kugelsegmentförmig ausgebildet ist und zwei zwischen den Seiten (34, 36) plan ausgebildete Führungsflächen (38, 40) aufweist, die ebenfalls sich gegenüberliegend angeordnet sind, und wobei das Zwischenelement (30) im wesentlichen ringförmig ausgebildet ist und in seinem das Lager (28) umgreifenden Bereich zwei sich gegenüberliegende Flachsegmente (42, 43) und zwei sich gegenüberliegende Kurvensegmente (44, 46) aufweist, derart, dass die Führungsflächen (38, 40) an an den Flachsegmenten (42, 43) ausgebildeten Gegenflächen (50, 52) zur Anlage kommen, so dass die Einfassung (32) im gelösten Zustand gegenüber dem Lager (28) In Richtung der Führungsflächen (38, 40) frei beweglich ist, während die Einfassung (32) im geklemmten Zustand über das Zwischenelement (30) klemmend am Lager (28) gehalten ist.

2. Verankerungselement nach Anspruch 1,
**dadurch gekennzeichnet,**
**dass** die Einfassung (32) starr mit der Halterung (10) und das Lager (28) starr mit dem Befestigungselement (14) verbunden ist.

3. Verankerungselement nach einem der vorangehenden Ansprüche,
**dadurch gekennzeichnet,**
**dass** die planen Gegenflächen (50, 52) an je einem Flachsegment (42, 43) des Zwischenelementes (30) ausgebildet sind, welche in Umfangsrichtung beabstandet von kugelsegmentförmigen Kurvensegmenten (44, 45) des Zwischenelementes (30) angeordnet sind.

4. Verankerungselement nach einem der vorangehenden Ansprüche,
**dadurch gekennzeichnet,**
**dass** die Führungsflächen und/oder die Gegenflächen geriffelt ausgebildet sind.

5. Verankerungselement nach einem der Ansprüche 1 bis 3,
**dadurch gekennzeichnet,**
**dass** die Führungsflächen und die Gegenflächen radiale angeordnete Nuten aufweisen, wobei die Nuten derart voneinander beabstandet sind, dass die Gegenfläche in gegenüberliegenden Nuten der Führungsfläche eingreift und umgekehrt.

6. Verankerungselement nach einem der Ansprüche 1 bis 3,
**dadurch gekennzeichnet,**
**dass** die Führungsflächen (30, 40) und/oder die Gegenflächen (50, 52) aufgeraut sind.

7. Verankerungselement nach einem der vorangehenden Ansprüche,
**dadurch gekennzeichnet,**
**dass** das Lager zumindest in seinem kugelsegmentförmigen Bereich und/oder in dem diesem Bereich zugewandten Halteflächen des Zwischenelementes aufgeraut ausgebildet ist/sind.

## Claims

1. An anchoring element for fastening a rod of a device for adjusting a human or animal vertebral column on a vertebra, said anchoring element having a retaining means (10) for receiving the rod, a securing element (26) that is attachable on the retaining means and acting against the rod, a fastening element (14) for attachment to the vertebral body and a clamping device (12) which is arranged between the retaining means (10) and the fastening element (14), including a ring-shaped mount (32), a bearing (28) and an intermediate element (30) embedded in the mount (32) and surrounding the bearing, said bearing (28) having the shape of a spherical segment on two opposing sides (34, 36) and comprising two guiding surfaces (38, 40) configured to be planar between the sides (34, 36) and also being oppositely disposed from each other, and said intermediate element (30) being substantially configured to be annular and comprising in its region surrounding the bearing (28) two opposing flat segments (42, 43) and two opposing curved segments (44, 45) so that the guiding surfaces (38, 40) abut onto counter surfaces (50, 52) formed on the flat segments (42, 43) so that the mount (32) is free to move toward the guiding surfaces (38, 40) relative to the bearing (28) when in its released state whilst the mount (32) is clampingly retained on the bearing (28) through the intermediate element (30) when in its clamped state.

2. The anchoring element as set forth in claim 1,
**characterized in**
**that** the mount (32) is rigidly connected to the retaining means (10) and the bearing (28) to the fastening element (14).

3. The anchoring element as set forth in any of the afore mentioned claims,
**characterized in**
**that** the level counter surfaces (50, 52) are formed on a respective one of the flat segments (42, 43) of the intermediate element (30), said flat segments being disposed in the circumferential direction at a distance from curve segments (44, 45) of the intermediate element (30) that are in the shape of a spherical segment.

4. The anchoring element as set forth in any of the afore mentioned claims,
**characterized in**
**that** the guiding surfaces and/or the counter surfaces are configured to be riffled.

5. The anchoring element as set forth in any of the claims 1 through 3,
**characterized in**
**that** the guiding surfaces and the counter surfaces comprise radially disposed grooves, said grooves being spaced apart in such a manner that the counter surface engages into opposite grooves of the guiding surface and vice versa.

6. The anchoring element as set forth in any of the claims 1 through 3,
**characterized in**
**that** the guiding surfaces (30, 40) and/or the counter surfaces (50, 52) are roughened.

7. The anchoring element as set forth in any of the afore mentioned claims,
**characterized in**
**that** the bearing is configured to be roughened, at least in its region which is in the shape of a spherical segment and/or in the retaining surfaces of the intermediate element that are turned toward said region.

## Revendications

1. Un élément d'ancrage destiné à fixer sur une vertèbre une tige d'un dispositif de réduction d'une colonne vertébrale humaine ou animale, avec une fixation (10) destinée à recevoir la tige, avec un élément de sécurisation (26) agissant contre la tige et apte à être monté sur la fixation, avec un élément de fixation (14) destiné à être monté sur la vertèbre et avec un dispositif de serrage (12) interposé entre la fixation (10) et l'élément de fixation (14) comprenant une bordure (32) annulaire, un palier (28) et un élément intermédiaire (30) noyé dans la bordure (32) et entourant le palier, le palier (28) étant réalisé en forme de segment sphérique sur deux faces (34, 36) situées en regard l'une de l'autre, et comprenant entre les faces (34, 36) deux surfaces de guidage (38, 40) planes, également situées en regard l'une de l'autre, l'élément intermédiaire (30) étant sensiblement réalisé en forme d'anneau et comprenant dans sa partie entourant le palier (28) deux segments plats (42, 43) se faisant face et deux segments courbes (44, 45) se faisant face, de telle sorte que les surfaces de guidage (38, 40) arrivent en appui contre les surfaces antagonistes (50, 52) formées sur les segments plats (42, 43), de sorte que la bordure (32) est librement mobile par rapport au palier (28) en direction des surfaces de guidage (38, 40) à l'état désengagé tandis que la bordure (32) est retenue par serrage sur le palier (28) par l'intermédiaire de l'élément intermédiaire (30) à l'état serré.

2. Elément d'ancrage selon la revendication 1,
**caractérisé en ce**
**que** la bordure (32) est reliée de façon rigide à la fixation (10) et que le palier (28) est relié de façon rigide à l'élément de fixation (14).

3. Elément d'ancrage selon l'une quelconque des revendications précédentes,
**caractérisé en ce**
**que** les surfaces antagonistes (50, 52) planes sont formées sur un segment plat (42, 43) respectif de l'élément intermédiaire (30) et sont disposées circonférentiellement à distance de segments courbes (44, 45) en forme de segments sphériques de l'élément intermédiaire (30).

4. Elément d'ancrage selon l'une quelconque des revendications précédentes,
**caractérisé en ce**
**que** les surfaces de guidage et/ou les surfaces antagonistes sont cannelées.

5. Elément d'ancrage selon l'une quelconque des revendications 1 à 3,
**caractérisé en ce**
**que** les surfaces de guidage et les surfaces antagonistes comportent des rainures disposées radialement, les rainures étant espacées de telle sorte l'une de l'autre que la surface antagoniste s'engage dans les rainures situées en regard de la surface de guidage et inversement.

6. Elément d'ancrage selon l'une quelconque des revendications 1 à 3,
**caractérisé en ce**
**que** les surfaces de guidage (30, 40) et/ou les surfaces antagonistes (50, 52) sont rendues rugueuses.

7. Elément d'ancrage selon l'une quelconque des revendications précédentes,
**caractérisé en ce**
**que** le palier, au moins dans sa partie en forme de segment sphérique, et/ou des surfaces de retenue de l'élément intermédiaire tournées vers cette partie est/sont rendus rugueux.
